(19) Europäisches Patentamt
European Patent Office
Office européen des brevets

(11) Veröffentlichungsnummer: **0 144 069**
**B1**

(12) # EUROPÄISCHE PATENTSCHRIFT

(45) Veröffentlichungstag der Patentschrift: **31.08.88**

(51) Int. Cl.⁴: **A 61 K 47/00,** C 07 C 43/13, C 07 C 43/178, C 07 C 69/30

(21) Anmeldenummer: **84114406.6**

(22) Anmeldetag: **28.11.84**

(54) **Arzneimittel mit verbesserter Penetration der Gewebsmembran.**

(30) Priorität: **01.12.83 DE 3343530**

(43) Veröffentlichungstag der Anmeldung:
**12.06.85 Patentblatt 85/24**

(45) Bekanntmachung des Hinweises auf die Patenterteilung:
**31.08.88 Patentblatt 88/35**

(84) Benannte Vertragsstaaten:
**AT BE CH DE FR GB IT LI LU NL SE**

(56) Entgegenhaltungen:
**EP-A-0 035 132**
**FR-A-1 599 577**
**NL-C-19 434**
**US-A-3 290 388**
**US-A-3 331 742**

**CHEMICAL ABSTRACTS, Band 96, Nr. 16, 19. April 1982, Seite 460, Nr. 129724a, Columbus, Ohio, US; K. UNNO u.a.: "Preparation and tissue distribution of 5-fluorouracil emulsion" & BYOIN YAKUGAKU 1980, 6(1), 14-20**
**CHEMICAL ABSTRACTS, Band 91, Nr. 7, 13. August 1979, Seite 646, Nr. 56320a, Columbus, Ohio, US; N.A. ARTAMONOVA u.a.: "Preparation and properties of monoglycerides of low-molecular-weight carboxylic acids" & IZV. AKAD. NAUK KAZ. SSR, SER. KHIM. 1979, (2), 35-7**
**CHEMICAL ABSTRACTS, Band 90, Nr. 3, 15. Januar**

(73) Patentinhaber: **Max- Planck- Gesellschaft zur Förderung der Wissenschaften e.V., Bunsenstrasse 10, D-3400 Göttingen (DE)**

(72) Erfinder: **Eibl, Hansjörg, Steinweg 51, D-3406 Bovenden (DE)**

(74) Vertreter: **Weickmann, Heinrich, Dipl.- Ing., Patentanwälte Dipl.- Ing. H.Weickmann Dipl.- Phys.Dr. K.Fincke Dipl.- Ing. F.A.Weickmann Dipl.- Chem. B. Huber Dr.- Ing. H. Liska Dipl.- Phys.Dr. J. Prechtel Postfach 860820, D-8000 München 86 (DE)**

(56) Entgegenhaltungen: (Fortsetzung)
**1979, Seite 581, Nr. 22245u, Columbus, Ohio, US; N.G. MARKINA u.a.: "Production of monoallyl ethers of glycerol" & NOUYE METODY SINTEZA KISLORODOSODERZH. SOEDIN. I MONOMEROV NA OSNOVE NEFT. SYR'YA 1978, 149-55**

**Die Akte enthält technische Angaben, die nach dem Eingang der Anmeldung eingereicht wurden und die nicht in dieser Patentschrift enthalten sind.**

EP 0 144 069 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Arzneimittel, welches eine verbesserte Penetration des Wirkstoffes durch die Gewebsmembran bzw. Schranke des Zielorgans ermöglicht.

Ein bekanntes Problem bei der Verabreichung von Arzneimitteln besteht darin, daß der eigentliche Wirkstoff häufig die Zellmembran nur schlecht passieren kann, so daß entweder die an sich möglichen Wirkungen des Arzneimittels in der Praxis nicht erzielt werden können oder der Wirkstoff so überdosiert werden muß, daß hierdurch die unerwünschten Nebenwirkungen, insbesondere in anderen Organen als dem Zielorgan, verstärkt werden.

Besonders problematisch in dieser Hinsicht ist die sogenannte Blut-Hirn-Schranke. Die normale Blut-Hirn-Schranke stellt eine hochselektive Permeabilitätsschranke dar, welche den Blut/Hirntransfer vieler Verbindungen behindert. Diese besonders ausgeprägte Schranke hat ihre anatomische Basis in den Kapillargefäßen, welche spezielle Strukturmerkmale aufweisen. Die Fähigkeit eines Wirkstoffs in freier Lösung (d. h. nicht an Protein gebunden) im Blutplasma die Blut-Hirn-Schranke zu penetrieren wird weitgehend durch die Fähigkeit des Wirkstoffes bestimmt, sich selbst aus dem Plasma abzuscheiden und in das Lipid der endothelialen Zellplasmamembranen einzudringen. Falls hier kein spezifischer Mechanismus vorliegt, ist die Lipidlöslichkeit der wesentliche Faktor, der das Penetrieren des Wirkstoffes durch die Blut-Hirn-Barriere bedingt, solange das Molekulargewicht des Wirkstoffes nicht größer als etwa 500 ist. Höhermolekulare Wirkstoffe vermögen die Blut-Hirn-Schranke auch dann nicht zu penetrieren wenn die Lipidlöslichkeit gut ist.

Es wurde daher bereits vorgeschlagen, Arzneimittel chemisch zu modifizieren durch Anhängen eines Restes mit hoher Lipidlöslichkeit, welche das Eindringen in die Schranke erleichtern. Bei geeigneter Wahl dieser Gruppe würde sie durch den Stoffwechsel dann wieder abgespalten, wobei der Wirkstoff in seiner aktiven Form freigesetzt wird.

Ein Nachteil dieses Konzepts besteht darin, daß eine unter Umständen schwierig durchzuführende Modifikation des eigentlichen Wirkstoffes erforderlich ist und bei der bekannten Empfindlichkeit der Wirksamkeit von Arzneimittelwirkstoffen gegen Veränderungen im Molekül Wirkungsverschlechterungen bzw. neue unerwünschte Nebenwirkungen zu befürchten sind.

Ähnliche Schwierigkeiten wie bei der Blut-Hirn-Schranke liegen auch bei anderen Organen vor, beispielsweise bei der Leber, Haut usw.

Der Erfindung liegt daher die Aufgabe zugrunde, dieses Problem auf einfache Weise und ohne Veränderung des eigentlichen Wirkstoffes zu lösen.

Erfindungsgemäß gelingt dies mit einem Arzneimittel, welches dadurch gekennzeichnet ist, daß es aus einem Wirkstoff in Kombination mit einer Verbindung der allgemeinen Formel

$H_2C - O - R_1$
|
$HC - O - R_2$
|
$H_2C - OH$

in der einer der Reste $R_1$ und $R_2$ eine Alkyl-, Alkenyl-, Alkinyl- oder Alkoylgruppe mit je 3 bis 7 C-Atomen und der andere Rest ein H-Atom bedeutet und üblichen pharmazeutischen Zusatz- und Verdünnungsmitteln besteht.

Die Verbindung der allgemeinen Formel ist ein Glycerinderivat, welches entweder in Stellung 1 oder in Stellung 2 substituiert ist mit einer der obengenannten kurzkettigen Gruppen. Die Substituenten können geradkettig oder verzweigt und gegebenenfalls auch cyclisch sein und bis zu zwei Doppel- oder Dreifachbindungen enthalten. Typische Beispiele für die erfindungsgemäßen Verbindungen sind 1-n-Propylglycerin, 1-n-Isopropylglycerin, 1-n-Butylglycerin, 1-Isobutylglycerin, 1-tert.-Butylglycerin, 1-n-Pentylglycerin, 1-n-Hexylglycerin, 1-n-Cyclohexylglycerin und 1-n-Heptylglycerin sowie deren Isomere 1-Methylbutylglycerin, 1-Allylglycerin, 1-Butinglycerin, die entsprechenden 2-Glycerinverbindungen sowie die Propionsäure-, Buttersäure-, Valeriansäure-, Valeriansäure, Önanthsäure, Acrylsäure-, Krotonsäure-, Angelikasäure- bzw. Tiglinsäure-, Hexensäure-, Heptensäure-, Propionsäure- und Tetrolsäureester in Position 1 oder 2 des Glycerins.

Aus der US-A-3 290 388 sind 1- und 2-Alkinyl-Glycerinderivate mit acarizider und insektizider Wirkung bekannt. Die FR-A-1 599 577 beschreibt ein verfahren zur Herstellung von 2-Alkyl-Glycerinderivaten, z. B. von 2-Propyl-Glycerin. Aus der EP-A-35 132 ist es bekannt, Glycerin mono- oder -di-niederalkylether als Lösungsmittel für pharmazeutische Zubereitungen zu verwenden.

Wesentlich beim erfindungsgemäßen Arzneimittel ist, daß der Wirkstoff und die Verbindung der allgemeinen Formel, bei der es sich um ein $C_1$- oder $C_2$-Glycerinderivat handelt, gemeinsam und gleichzeitig zur Anwendung kommen und zwar durch Injektion in ein Blutgefäß, welches das Arzneimittel dem Zielorgan unmittelbar und auf möglichst kurzem Wege zuführt. Ist das Zielorgan des Arzneimittels das Gehirn, so eignet sich für die Applikation z. B. die A. Carotis. Bei anderen Organen gilt sinngemäß dasselbe. Beispielsweise eignet sich für Verabreichung im Oberschenkel die Femoralisarterie.

Überraschenderweise wurde gefunden, daß das erfindungsgemäße Arzneimittel innerhalb von Sekunden

nach der Applikation einen außerordentlich starken Anstieg der Wirkstoffkonzentration im Zielorgan zur Folge hat. Es wird angenommen, daß der im erfindungsgemäßen Arzneimittel enthaltene Zusatzstoff, das Glycerinderivat der allgemeinen Formel, die Gewebsschranke, beispielsweise die Blut-Hirn-Schranke, kurzzeitig öffnet und in dieser Zeit dem eigentlichen Wirkstoff des Arzneimittels den Zutritt zum Zielorgan ermöglicht. Die Dauer dieses Effekts ist kurz und in Abhängigkeit von der Konzentration des Glycerinderivates auf höchstens etwa eine Minute anzusetzen. Danach erfolgt kein merklicher weiterer Anstieg der Wirkstoffkonzentration im Zielorgan mehr. Innerhalb des genannten kurzen Zeitraums gelingt es jedoch erfindungsgemäß, die Dosierung des eigentlichen Wirkstoffes jenseits der Membranbarriere um ein vielfaches zu steigern.

Die Wirksamkeit des erfindungsgemäßen Arzneimittels wurde nach der Methode von Oldendorf (Brain-Res. 24, 372-376 (1970)) getestet. Bei dieser Methode werden die eingesetzten Wirkstoffe in radioaktiv markierter Form verabreicht und dann nach vorgegebener Zeit die in das Zielorgan übergegangene Radioaktivität bestimmt. Beim erfindungsgemäßen Arzneimittel erfolgte die Verabreichung in die A. Carotis. 15 Sekunden später wurde das Versuchstier dekapitiert, das Hirn entnommen und die darin gebundene Radioaktivität bestimmt. In der nachstehenden Tabelle 1 sind die mit diesem Verfahren erhaltenen Ergebnisse wiedergegeben und zwar für 11 verschiedene Verbindungen, von denen 9 Antitumormittel sind und zwar bei Verabreichung ohne Zusatz einer Verbindung der allgemeinen Formel, mit Zusatz von Glycerin-1-propylether und Glycerin-1-pentylether. Die Zahlenwerte geben den prozentualen Anteil an Wirkstoff an, welcher die Blut-Hirn-Schranke passiert hat. Als Vergleichssubstanz wurde eine isotane Pufferlösung eingesetzt. Die Verbindung der allgemeinen Formel lag in isoosmolarer Konzentration vor, was etwa 0,3 Osmol/l in der verabreichten Lösung entspricht.

**Tabelle 1**

| Substanz: | ohne | mit $C_3$ | mit $C_5$ |
|---|---|---|---|
| Endoxan | 11,4 ± 0,4 | 18,9 ± 0,3 | 53,5 ± 6,1 |
| Daunomycin | 10,8 ± 2,7 | 24,1 ± 3,3 | 76,8 ± 7,0 |
| Methotrexat | 4,7 ± 0,6 | 5,7 ± 1,0 | 26,2 ± 5,8 |
| Vinblastin | 5,4 ± 1,7 | 4,2 ± 0,6 | 44,3 ± 6,7 |
| Bleomycin | 4,9 ± 1,4 | ./. | 5,7 ± 1,8 |
| Peplomycin | 4,5 ± 1,9 | ./. | 13,8 ± 1,5 |
| 5-Fluorouracil | 3,7 ± 1,0 | ./. | 13,0 ± 0,1 |
| Vepesid | 3,8 ± 0,5 | ./. | 15,2 ± 2,1 |
| ET 18-O-$CH_3$* | 4,5 ± 0,8 | 16,4 ± 4,1 | ./. |
| Glycerin | 3,9 ± 1,9 | 11,9 ± 2,9 | 27,4 ± 2,7 |
| Phosphatidyl-Cholin* | 2,6 ± 0,8 | ./. | 16,3 ± 3,3 |
| Mitoxandrone | 1,8 ± 0,1 | ./. | 25,1 ± 2.0 |

Analoge Versuche wurden mit den in Stellung 2 des Glycerins substituierten Verbindungen durchgeführt und ergaben vergleichbare Resultate.

* in Ringeralbumin gelöst

Die Wirkstoffe wurden bei den Versuchen nach der Methode von Oldendorf in Mengen zwischen 10 und 100 µmol eingesetzt. Dabei ergab sich keine Abhängigkeit des Penetrationsprozentsatzes von der Wirkstoffkonzentration, d. h. daß mit zunehmender Wirkstoffmenge der die Blut-Hirn-Schranke passierende Prozentsatz etwa gleich blieb. Bei Methotrexat als Wirkstoff wurde die Penetration auch bei höheren Konzentrationen untersucht. Hier ergab sich, daß bis über 10 mMol eine lineare Zunahme des an den Wirkungsort vorgedrungenen Wirkstoffanteils erfolgt mit praktisch gleichbleibendem prozentualem Anteil an die Schranke passierendem Wirkstoff.

Die erreichte Penetration hängt auch von der eingesetzten Menge an Glycerinderivat der allgemeinen Formel ab. Bezogen auf die in Tabelle 1 angegebenen Ergebnisse wurde z. B. mit Endoxan und dem $C_3$-Zusatzmittel eine Penetration von knapp 20 % erzielt, während mit einer gleichen Menge des $C_5$-Zusatzmittels die Penetration über 50 % liegt. Erhöht man jedoch die Menge des $C_3$-Derivates auf das 2,5-fache, so erhöht sich auch der penetrierte Prozentsatz auf etwa 50 %.

Analoge Versuche wurden auch mit anderen Organen durchgeführt und ergaben völlig vergleichbare Ergebnisse. So wurde beispielsweise bei einer Rattenextremität das Arzneimittel in die Femoralis verabreicht und die Extremität nach 30 Sekunden amputiert und in mehrere Teile geteilt. Dabei zeigte sich, daß die die Membran passierende Konzentration mit zunehmender Entfernung vom Verabreichungsort abnahm, jedoch auch in den am weitesten entfernten Teilen noch signifikant erhöht war.

Die im erfindungsgemäßen Arzneimittel enthaltene Zusatzverbindung gemäß allgemeiner Formel erwies sich als völlig untoxisch. Die fehlende Toxizität der Verbindungen der allgemeinen Formel zeigt sich beispielsweise daraus, daß diese in 50 %-iger Konzentration drei Wochen lang an Ratten ip verabreicht wurden, ohne daß irgendwelche nachteiligen Wirkungen festgestellt werden konnten. Im Eigenversuch wurden die $C_3$-

Verbindungen subcutan in 17 %-iger Konzentration verabreicht, ohne irgendwelche negativen Erscheinungen.

Als zweckmäßige Obergrenze dürfte bei den Derivaten mit einem 3 Kohlenstoffatome enthaltenden Rest eine Konzentration, die einer 30 %-igen Lösung entspricht, anzusehen sein. Bei den $C_5$-Derivaten liegt die zweckmäßige Obergrenze im Bereich von 5 bis 8 % der zu injizierenden Lösung. Bei diesen Konzentrationen beträgt die Öffnungsdauer, bezogen auf die Blut-Hirn-Schranke etwa eine Minute.

Die Öffnungszeiten lassen sich bestimmen, indem man zuerst die Verbindung der allgemeinen Formel spritzt und dann zu verschiedenen Zeiten bis zu einer Minute danach den Wirkstoff verabreicht. Ist der Abstand zwischen den beiden Injektionen größer als die Öffnungsdauer, so hat sich die Membranbarriere wieder geschlossen und der aufgenommene Prozentsatz an Wirkstoff ist entsprechend niedrig.

Die Verabreichung erfolgt zweckmäßigerweise als "Bolusinjektion", wobei anfangs keine wesentliche Mischung der injizierten Substanz mit dem Blut erfolgt.

Die im erfindungsgemäßen Arzneimittel enthaltenen Zusatzverbindungen der allgemeinen Formel werden von der Leber rasch abgebaut und sind daher innerhalb kürzester Zeit im Kreislauf nicht mehr nachweisbar. Dies ist insbesondere bei den Verbindungen der allgemeinen Formel, in denen $R_1 = H$ ist, überraschend, da ein die 2-O-Alkylposition spaltendes Enzym bisher nicht beschrieben wurde.

Die Auswahl des Alkyl-Glycerin-Bestandteils gemäß allgemeiner Formel für das erfindungsgemäße Arzneimittel hängt in gewissem Ausmaß von den Eigenschaften des eigentlichen Wirkstoffes ab. Hat der eigentliche Wirkstoff keine oberflächenaktiven Eigenschaften, so wurden die besten Ergebnisse dann erzielt, wenn $R_1$ oder $R_2$ in der Verbindung der allgemeinen Formel 5 bis 7 C-Atome aufweist. Besitzt der Wirkstoff hingegen oberflächenaktive Eigenschaften, so erzielt man die besten Resultate mit einer Verbindung der allgemeinen Formel, in der $R_1$ oder $R_2$ 3 bis 5 C-Atome aufweist.

Das erfindungsgemäße Arzneimittel ist hinsichtlich der Auswahl des Wirkstoffes an sich keinen Begrenzungen unterworfen, d. h. alle üblichen Wirkstoffe mit ungenügender Penetration in das Zielorgan können durch die erfindungsgemäße Zubereitung verbessert werden. Es hat sich jedoch herausgestellt, daß im allgemeinen eine deutliche Wirksamkeit nur bei Wirkstoffen mit einem Molekulargewicht im Bereich von etwa 100 bis etwa 3000 gegeben ist und die besten Ergebnisse mit solchen Wirkstoffen erzielt werden, deren Molekulargewicht unter 2000, insbesondere im Bereich von 200 bis 1500 liegt.

Einen Anhaltspunkt für die Brauchbarkeit von Wirkstoffen im Rahmen des erfindungsgemäßen Arzneimittels gibt ihr Wasser/Öl-Verteilungskoeffizient. In der beigefügten Zeichnung ist

Fig. 1 eine graphische Darstellung, in welcher der die Blut-Hirn-Schranke passierende Prozentsatz gegen den Logarithmus des Wasser/Öl-Verteilungskoeffizienten für die Wirkstoffe 5-Fluorouracil (1), Methotrexat (2), Endoxan (3) und Daunomycin, also Wirkstoffe mit einem Molekulargewicht bis 600, aufgetragen ist. Man erkennt, daß auch bei einem Logarithmus -3 zwar noch eine wesentliche Verbesserung erzielt wird, jedoch mit zunehmender Lipophilie die Penetration etwa linear mit dem Logarithmus des Wasser/Öl-Verteilungskoeffizienten zunimmt.

Fig. 2 eine graphische Darstellung analog Fig. 1 für Wirkstoffe mit einem über 600 liegenden Molekulargewicht und zwar Bleomycin (5), Vepesid bzw. Peplomycin (6, 7) und Vinblastin (8). Man erkennt, daß bei Bleomycin mit stark hydrophilem Charakter die Penetrationsverbesserung relativ gering ist, während sie bei dem lipophilsten dieser Wirkstoffe, dem Vinblastin auf mehr als das 25fache gesteigert wird (nach Abzug des methodisch bedingten Leerwertes von etwa 3 % für Sucose).

Da die Blut-Hirn-Schranke das für die Chemotherapie größte Hindernis unter dem Gesichtspunkt der Membranpenetration darstellt, eignet sich das erfindungsgemäße Arzneimittel insbesondere für Wirkstoffe, die im Hirn angreifen sollen. Dies sind insbesondere Cytostatika, beispielsweise jedoch auch Psychopharmaka, Mittel gegen Parkinson'sche Krankheit (Dopamin) und andere.

Durch die Verwendung von Mischungen von Verbindungen der allgemeinen Formel lassen sich dabei für jeden Wirkstoff bestimmte gewünschte Bedingungen einstellen, was sich nach der Methode von Oldendorf leicht kontrollieren läßt.

Das erfindungsgemäße Arzneimittel ermöglicht es daher, Wirkstoffe in höherer Konzentration als bisher in das Zielorgan zu bringen bzw. gleiche Effekte mit wesentlich geringerer Wirkstoffmenge zu erreichen.

Die folgenden Beispiele erläutern die Erfindung weiter.

**Beispiel 1**

Herstellung der 1-Alkyl-glycerine

Käufliches 1,2-Isopropyliden-glycerin, 0,2 Mol, wird in 300 ml tert.-Butanol gelöst und mit 0,3 Mol K-tert.-Butylat versetzt. Man kocht unter Rückfluß und versetzt tropfenweise über einen Zeitraum von 60 Minuten mit einer Lösung aus Alkylbromid, 0,25 Mol, in 100 ml THF. Danach wird weitere 60 Minuten unter Rückfluß gekocht. Man kühlt ab, versetzt mit 300 ml Diisopropylether und 300 ml Wasser. Die obere Phase wird einrotiert, der ölige Rückstand in 500 ml $CH_3OH$ aufgenommen, mit 50 ml 1N-HCl versetzt und unter Rückfluß

gekocht. Nach 60 Minuten ist die Abspaltung der Schutzgruppen vollständig. Man neutralisiert mit $Na_2CO_3$, 0,1 Mol, unten Rühren, filtriert und entfernt die Lösungsmittel im Wasserstrahlvakuum. Der ölige Rückstand wird destilliert. Die physikalischen Daten zeigt Tabelle 2. Die Ausbeuten an reinen 1-Alkylglycerinen liegen zwischen 80 und 90 % bezogen auf 1,2-Isopropyliden-glycerin.

**Tabelle 2**

| 1-Alkylglycerine | kp | $n^{20}_D$ °C |
|---|---|---|
| Propyl | 83-84° C/6,5 Pa ( 0,05 mm) | 1.4420 |
| Butyl | 124° C/1300 Pa (10 mm) | 1.4444 |
| Pentyl | 134° C/1300 Pa (10 mm) | 1.4500 |
| Hexyl | 141° C/1300 Pa (10 mm) | 1.4511 |
| Heptyl | 147° C/1300 Pa (10 mm) | 1.4525 |

**Beispiel 2**

Herstellung der 2-Alkyl-glycerine

Ausgehend von 1,3-Benzyliden-glycerin dargestellt nach Johary and Owen (J. Chem. Soc., 1955, 1299-1301) wurden die Alkylreste in die 2-Position eingeführt. Dazu wurde 1,3-Benzyliden-Glycerin, 0,2 Mol, in 300 ml tert.-Butanol gelöst, mit K-tert.-Butylat, 0,3 Mol versetzt und unter Rückfluß gekocht. Nach Eintropfen von Alkylbromid, 0,25 Mol, in 100 ml THF in einem Zeitraum von 60 Minuten wird noch weitere 60 Minuten unter Rückfluß erhitzt. Man kühlt, versetzt mit 300 ml Diisopropylether und schüttelt gegen 300 ml Wasser aus. Die obere Phase wird einrotiert, in 500 ml Methanol gelöst und mit 50 ml 1N-HCl unter Rückfluß 30 Minuten erhitzt. Man kühlt, neutralisiert mit $Na_2CO_3$, 0,1 Mol, unter Rühren und filtriert. Nach Entfernung der Lösungsmittel im Wasserstrahlvakuum wird der ölige Rückstand destilliert. Die physikalischen Daten zeigt Tabelle 3. Die Ausbeuten an reinen 2-Alkylglycerinen liegen zwischen 70 und 80 % bezogen auf 1,3-Benzyliden-glycerin.

**Tabelle 3**

| 2-Alkylglycerine | Kp | $n^{20}_D$ °C |
|---|---|---|
| Propyl | 126° C/1300 Pa (10 mm) | 1.5000 |
| Butyl | 134° C/1300 Pa (10 mm) | 1.4525 |
| Pentyl | 143° C/1300 Pa (10 mm) | 1,4554 |
| Hexyl | 150° C/1300 Pa (10 mm) | 1,4571 |
| Heptyl | 156° C/1300 Pa (10 mm) | 1,4589 |

**Patentansprüche** für die Vertragsstaaten: BE, CH, DE, FR, GB, IT, LU, NL, SE

1. Arzneimittel mit verbesserter Penetration der Zellmembran des Zielorgans, dadurch gekennzeichnet, daß es aus einem Wirkstoff in Kombination mit einer Verbindung der allgemeinen Formel

$$\begin{array}{l} H_2C - O - R_1 \\ \quad | \\ H\text{-}C - O - R_2 \\ \quad | \\ H_2C - OH \end{array}$$

2. Arzneimittel nach Anspruch 1, dadurch gekennzeichnet, daß $R_1$ oder $R_2$ 5 bis 7 C-Atome aufweist, wenn der Wirkstoff nicht oberflächenaktiv ist.

3. Arzneimittel nach Anspruch 1, dadurch gekennzeichnet, daß $R_1$ oder $R_2$ 3 bis 5 C-Atome aufweist, wenn der Wirkstoff oberflächenaktiv ist.

4. Arzneimittel nach einem der Ansprüche 1 bis 3, dadurch gekennzeichnet, daß der Wirkstoff ein Molekulargewicht im Bereich von 100 bis 3000 aufweist.

5. Arzneimittel nach Anspruch 4, dadurch gekennzeichnet, daß der Wirkstoff ein Molekulargewicht im Bereich von 200 bis 1500 aufweist.

6. Arzneimittel nach einem der vorhergehenden Ansprüche, dadurch gekennzeichnet, daß der Logarithmus des Wasser/Öl-Verteilungskoeffizienten des Wirkstoffes -3,0 oder größer ist.

7. Arzneimittel nach einem der vorhergehenden Ansprüche, dadurch gekennzeichnet, daß die Verbindung

der allgemeinen Formel für $R_1$ oder $R_2$ mit 3 bis 5 C-Atomen in isoosmolarer Konzentration vorliegt.

8. Arzneimittel nach einem der vorhergehenden Ansprüche, dadurch gekennzeichnet, daß es als Wirkstoff Endoxan, Daunomycin, Metotrexat, Vinblastin, Bleomycin, Peplomycin, 5-Fluorouracil, Mitoxandrone, Vepesit, ET 18-O-$CH_3$, Phosphatidylcholin oder Dopamin enthält.

**Patentansprüche** für den Vertragsstaat: AT

1. Verfahren zur Herstellung eines Arzneimittels mit verbesserter Penetration der Zellmembran des Zielorgans, dadurch gekennzeichnet, daß man den Wirkstoff mit einer Verbindung der allgemeinen Formel

$H_2C - O - R_1$
|
$H-C - O - R_2$
|
$H_2C - OH$

in der einer der Reste $R_1$ und $R_2$ eine Alkyl-, Alkenyl-, Alkinyl- oder Alkoylgruppe mit je 3 bis 7 C-Atomen und der andere Rest ein H-Atom bedeutet und üblichen pharmazeutischen Zusatz- und Verdünnungsmitteln besteht, kombiniert.

2. Verfahren nach Anspruch 1, dadurch gekennzeichnet, daß man eine Verbindung der allgemeinen Formel verwendet, in der $R_1$ oder $R_2$ 5 bis 7 C-Atome aufweist, wenn der Wirkstoff nicht oberflächenaktiv ist.

3. Verfahren nach Anspruch 1, dadurch gekennzeichnet, daß man einen Wirkstoff der allgemeinen Formel, in dem $R_1$ oder $R_2$ 3 bis 5 C-Atome aufweist, verwendet, wenn der Wirkstoff oberflächenaktiv ist.

4. Verfahren nach einem der Ansprüche 1 bis 3, dadurch gekennzeichnet, daß man einen Wirkstoff verwendet, der ein Molekulargewicht im Bereich von 100 bis 3000 aufweist.

5. Verfahren nach Anspruch 4, dadurch gekennzeichnet, daß man einen Wirkstoff verwendet, der ein Molekulargewicht im Bereich von 200 bis 1500 aufweist.

6. Verfahren nach einem der vorhergehenden Ansprüche, dadurch gekennzeichnet, daß man einen Wirkstoff verwendet, bei dem der Logarithmus des Wasser/Öl-Verteilungskoeffizienten -3,0 oder größer ist.

7. Verfahren nach einem der vorhergehenden Ansprüche, dadurch gekennzeichnet, daß man die Verbindung der allgemeinen Formel für $R_1$ oder $R_2$ mit 3 bis 5 C-Atomen in isoosmolarer Konzentration verwendet.

8. Verfahren nach einem der vorhergehenden Ansprüche, dadurch gekennzeichnet, daß man als Wirkstoff Endoxan, Daunomycin, Metotrexat, Vinblastin, Bleomycin, Peplomycin, 5-Fluorouracil, Mitoxandrone, Vepesit, ET 18-O-$CH_3$, Phosphatidylcholin oder Dopamin verwendet.

**Claims** for the contracting states BE, CH, DE, FR, GB, IT, LI, LU, NL, SE

1. Medicament with improved penetration of the cell membranes of object organ, characterised in that it consists of an active material in combination with a compound of the general formula

$H_2C - O - R_1$
|
$H-C - O - R_2$
|
$H_2C - OH$

in which one of the residues $R_1$ and $R_2$ signifies an alkyl, alkenyl, alkynyl or alkoyl group with, in each case, 3 to 7 C-atoms and the other residue signifies an H-atom, and conventional pharmaceutical additive and dilution agents.

2. Medicament according to claim 1, characterised in that $R_1$ or $R_2$ has 5 to 7 C-atoms when the active material is not surface-active.

3. Medicament according to claim 1, characterized in that $R_1$ or $R_2$ has 3 to 5 C-atoms when the active material is surface-active.

4. Medicament according to one of claims 1 to 3, characterized in that the active material has a molecular weight in the range of 100 to 3000.

5. Medicament according to claim 4, characterized in that the active material has a molecular weight in the range of 200 to 1500.

6. Medicament according to one of the preceding claims, characterized in that the logarithm of the water/oil partition coefficient of the active material is -3.0 or greater.

7. Medicament according to one of the preceding claims, characterised in that the compound of the general

formula for $R_1$ or $R_2$ with 3 to 5 C-atoms is present in isoosmolar concentration.

8. Medicament according to one of the preceding claims, characterised in that, as active material, it contains, endoxan, daunomycin, metotrexate, vinblastin, bleomycin, peplomycin, 5-flourouracil, mitoxandrone, vepesite, ET 18-O-$CH_3$, phosphatidyl choline or dopamine.

**Claims** for the contracting state: AT

1. Process for the preparation of a medicament with improved penetration of the cell membrane of the object organ, characterised in that one combines the active material with a compound of the general formula

$$\begin{array}{l} H_2C - O - R_1 \\ \quad | \\ H\text{-}C - O - R_2 \\ \quad | \\ H_2C - OH \end{array}$$

in which one of the residues $R_1$ and $R_2$ signifies an alkyl, alkenyl, alkynyl or alkoyl group with, in each case, 3 to 7 C-atoms and the other residue signifies an H-atom, and conventional pharmaceutical additive and dilution agents.

2. Process according to claim 1, characterised in that one uses a compound of the general formula, in which $R_1$ or $R_2$ has 5 to 7 C-atoms when the active material is not surface active.

3. Process according to claim characterized in that one uses a compound of the general formula, in which $R_1$ or $R_2$ has 3 to 5 C-atoms when the active material is surface-active.

4. Process according to one of claims 1 to 3, characterised in that one uses an active material which has a molecular weight in the range of 100 to 3000.

5. Process according to claim 4, characterized in that one uses an active material which has a molecular weight in the range of 200 to 1500.

6. Process according to one of the preceding claims, characterised in that one uses an active material in which the logarithm of the water/oil partition coefficient is -3.0 or greater.

7. Process according to one of the preceding claims, characterised in that the compound of the general formula for $R_1$ or $R_2$ with 3 to 5 C-atoms is used in isoosmolar concentration.

8. Process according to one of the preceding claims, characterised in thats active material, one uses endoxan, daunomycin, metotrexate, vinblastin, bleomycin, peplomycin, 5-fluorouracil, mitoxandrone, vepesite, ET 10-O-$CH_3$, phosphatidylcholine or dopamine.

**Revendications** pour les Etats contractants: BE, CH, LI, DE, FR, GB, IT, LU, NL, SE

1. Médicament ayant une pénétration améliorée de la membrane cellulaire de l'organe cible, caractérisé en ce qu'il se compose d'un principe actif associé à un composé répondant à la formule générale

$$\begin{array}{l} H_2C - O - R_1 \\ \quad | \\ HC - O - R_2 \\ \quad | \\ H_2C - O\ OH \end{array}$$

dans laquelle un des radicaux $R_1$ et $R_2$ désigne un groupe alkyle, alcényle, alcinyle ou alcoyle en $C_3$ à $C_7$ et l'autre radical désigne un atome H, et d'additifs et de diluants pharmaceutiques usuels.

2. Médicament suivant la revendication 1, caractérisé en ce que $R_1$ ou $R_2$ contient 5 à 7 atomes de carbone lorsque le principe actif n'est pas tensio-actif.

3. Médicament suivant la revendication 1, caractérisé en ce que $R_1$ ou $R_2$ contient 3 à 5 atomes de C lorsque le principe actif est tensio-actif.

4. Médicament suivant l'une des revendications 1 à 3, caractérisé en ce que le principe actif présente une masse moléculaire dans le domaine de 100 à 3000.

5. Médicament suivant la revendication 4, caractérisé en ce que le principe actif présente une masse moléculaire dans le domaine de 200 à 1500.

6. Médicament suivant l'une des revendications précédentes, caractérisé en ce que le logarithme du coefficient de répartition eau/huile du principe actif est de -3,0 ou davantage.

7. Médicament suivant l'une des revendications précédentes, caractérisé en ce que le composé répondant à

la formule générale, pour $R_1$ ou $R_2$ en $C_3$ à $C_5$, est présent à une concentration iso-osmolaire.

8. Médicament suivant l'une des revendications précédentes, caractérisé en ce qu'il contient comme principe actif de l'endoxane, de la daunomycine, du métotrexate, de la vinblastine, de la bléomycine, de la péplomycine, du 5-fluorouracile, de la mitoxandrone, de la vépésite, de l'ET 18-O-$CH_3$, de la phosphatidylcholine, ou de la dopamine.

**Revendications** pour l'Etat contractant: AT

1. Procédé de préparation d'un médicament ayant une pénétration améliorée de la membrane cellulaire de l'organe cible, caractérisé en ce qu'il se compose d'un principe actif associé à un composé répondant à la formule générale

$$\begin{array}{l} H_2C - O - R_1 \\ \quad | \\ HC - O - R_2 \\ \quad | \\ H_2C - OH \end{array}$$

dans laquelle un des radicaux $R_1$ et $R_2$ désigne un groupe alkyle, alcényle, alcinyle ou alcoyle en $C_3$ à $C_7$ et l'autre radical désigne un atome H, et d'additifs et de diluants pharmaceutiques usuels.

2. Procédé suivant la revendication 1, caractérisé en ce qu'on utilise un composé répondant à la formule générale, dans lequel $R_1$ ou $R_2$ contient 5 à 7 atomes de carbone, lorsque le principe actif n'est pas tensio-actif.

3. Procédé suivant la revendication 1, caractérisé en ce que l'on utilise un composé répondant à la formule générale, dans lequel $R_1$ ou $R_2$ contient 3 à 5 atomes de C, lorsque le principe actif est tensio-actif.

4. Procédé suivant l'une des revendications 1 à 3, caractérisé en ce qu'on utilise un principe actif qui présente une masse moléculaire dans le domaine de 100 à 3000.

5. Procédé suivant la revendication 4, caractérisé en ce qu'on utilise un principe actif qui présente une masse moléculaire dans le domaine de 200 à 1500.

6. Procédé suivant l'une des revendications précédentes, caractérisé en ce qu'on utilise un principe actif pour lequel le logarithme du coefficient de répartition eau/huile est de -3,0 ou davantage.

7. Procédé suivant l'une des revendications précédentes, caractérisé en ce que le composé répondant à la formule générale, pour $R_1$ ou $R_2$ en $C_3$ à $C_5$ est présent à une concentration iso-osmolaire.

8. Procédé suivant l'une des revendications précédentes, caractérisé en ce qu'on utilise comme principe actif l'endoxane, la daunomycine, le métotrexate, la vinblastine, la bléomycine, la péplomycine, le 5-fluorouracile, la mitoxandrone, la vépésite, l'ET 18-O-$CH_3$, la phosphatidylcholine ou la dopamine.

FIG.1

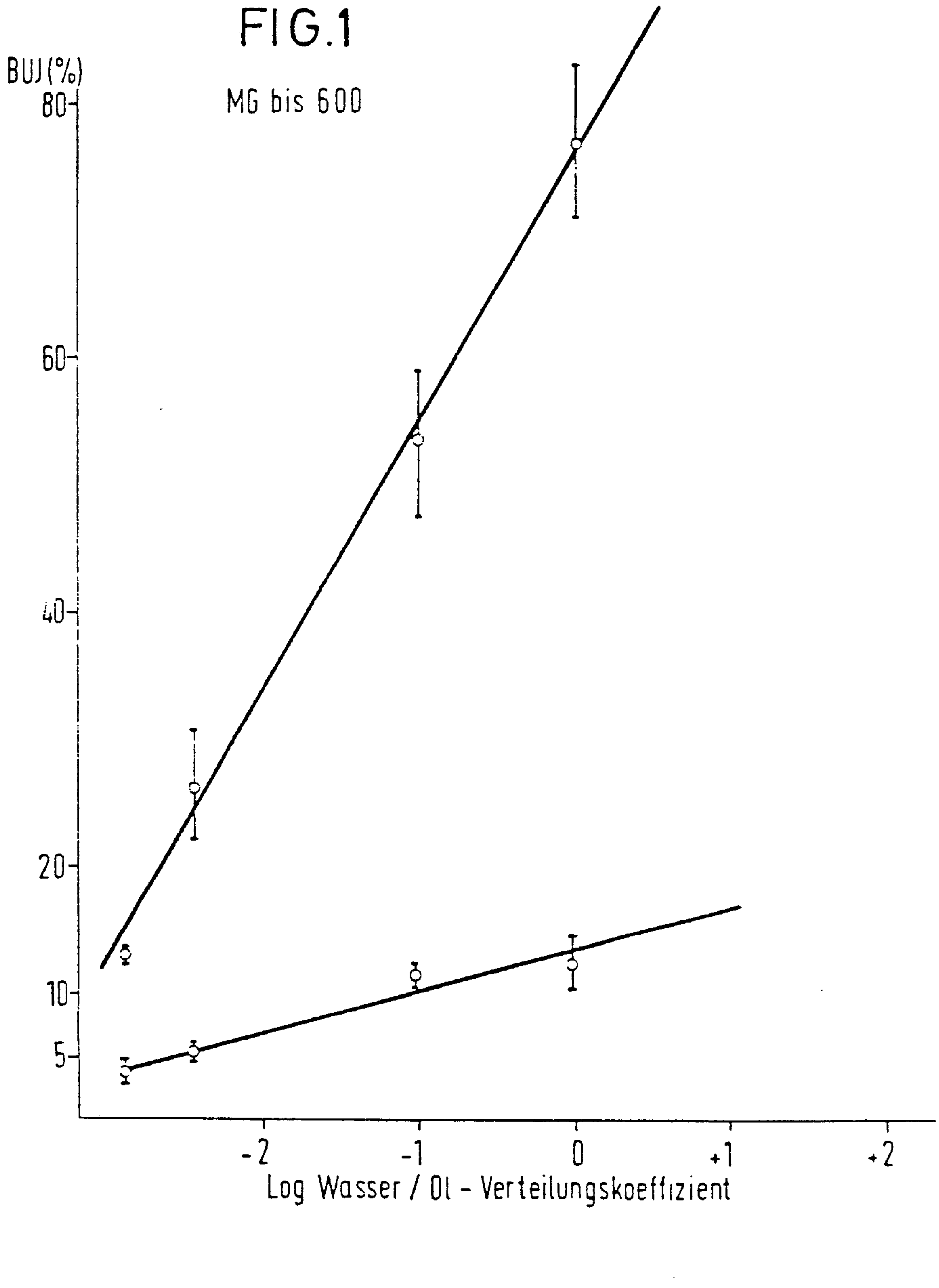
BUJ (%)
MG bis 600
80
60
40
20
10
5
-2
-1
0
+1
+2
Log Wasser / Öl - Verteilungskoeffizient

FIG. 2

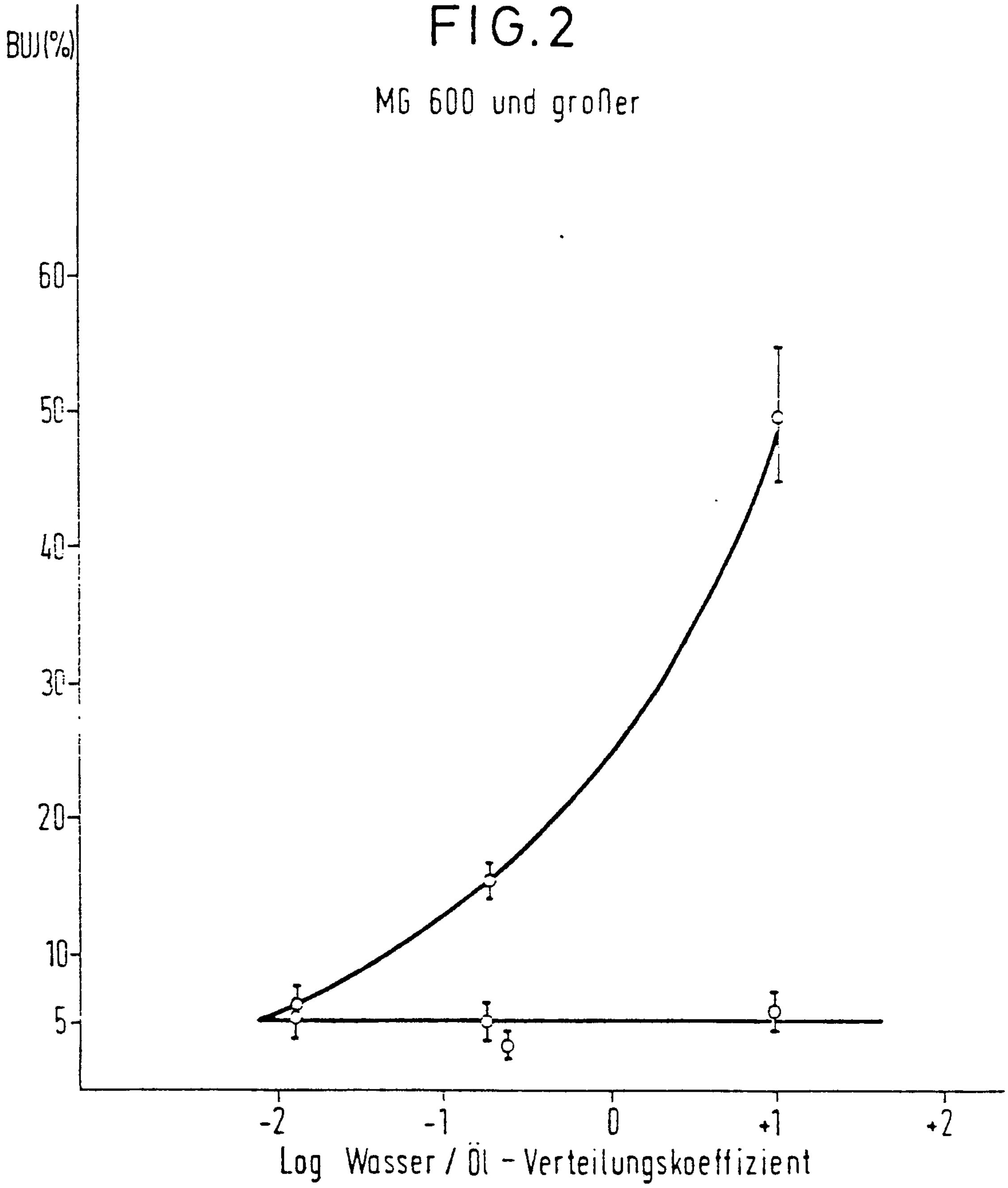
BUJ(%)
MG 600 und großer
60
50
40
30
20
10
5
-2
-1
0
+1
+2
Log Wasser / Öl - Verteilungskoeffizient